# EUROPEAN PATENT APPLICATION

(11) **EP 3 238 627 A1**
(43) Date of publication of application: **01.11.2017**
(21) Application number: 15873222.2
(22) Date of filing: 24.12.2015
(51) Int. Cl.: A61B 6/04, A61B 6/03, A61N 5/10

(54) **HOLDING DEVICE**

(30) Priority: 27.12.2014 JP 2014266898
(71) Applicant: Fujidenolo Co., Ltd., Komaki-shi, Aichi 485-0053 (JP); Medipolis Medical Research Institute, Ibusuki-shi, Kagoshima 891-0304 (JP)
(72) Inventor: ARIMURA Takeshi, Ibusuki-shi Kagoshima 891-0304 (JP); MATSUYAMA Mitsugi, Ibusuki-shi Kagoshima 891-0304 (JP); OGINO Takashi, Ibusuki-shi Kagoshima 891-0304 (JP); HISHIKAWA Yoshio, Ibusuki-shi Kagoshima 891-0304 (JP); NOMA Keiichi, Komaki-shi Aichi 485-0053 (JP); SAKURAGI Akari, Komaki-shi Aichi 485-0053 (JP); TAKEUCHI Norihide, Komaki-shi Aichi 485-0053 (JP)
(74) Representative: Kuhnen & Wacker
(86) International application number: PCT/JP2015/086114
(87) International publication number: WO 2016/104654

(57) **Abstract**

The objective of the invention is to provide a holding device which holds part of the body of a subject sufficiently securely. This holding device is provided with: a holding tool 16 which is provided with a curved surface 16a formed to match the shape of the body surface of a part of the body of a subject 8, and which is provided, on the curved surface 16a side thereof, with an adhesive layer 20 having adhesion properties with respect to the surface of the skin of the subject 8; an elongate arm member 18 to one end portion of which the holding tool 16 is attached; and a securing tool 14 for securing the arm member 18 to a bed 12, said securing tool 14 being attached to the end portion of the arm member 18 at the opposite end to the end portion to which the holding tool 16 is attached. By this means, even when carrying out treatment of a site which is liable to deform, such as a breast 8b, said site can be held sufficiently securely by affixing the holding tool 16 to the site using the adhesive layer 20. In other words, it is possible to provide a holding device 10 which holds part of the body of the subject 8 sufficiently securely.

## Description

### TECHNICAL FIELD

The present invention relates to a holding device holding a body part of a subject.

### BACKGROUND ART

For example, a device is known that fixes a body of a subject to a bed when X-ray imaging or radiation treatment is performed by a medical device. For example, a subject fixing device of a medical device described in Patent Document 1 is an example thereof. According to this technique, a top plate of bed for placing a subject thereon includes a bag body surrounding the subject, and the bag body is made up of a skin formed of a hard strong material and an expansion chamber formed of a soft elastic material inside thereof, and it is described that by injecting a gas or a liquid into the expansion chamber by a compressor, the subject can securely be fixed to the top plate of the bed in the state of being surrounded by the bag body.

### PRIOR ART DOCUMENT

### Patent Document

Patent Document 1: Japanese Laid-Open Patent Publication No. 2007-301217

### SUMMARY OF THE INVENTION

### Problem to Be Solved by the Invention

However, depending on a part of a subject's body to be treated, the part cannot necessarily be held sufficiently securely with the conventional technique in some cases. For example, considering treatment to the breast, the breast easily deforms due to gravity in accordance with a posture etc. of the subject, and it is therefore difficult for the conventional technique to sufficiently securely hold the breast while maintaining the shape thereof at the time of treatment. Furthermore, when it is required to keep a part such as the breast to be treated in substantially the same shape in a plurality of treatments performed at a time interval, the conventional technique may cause the shape of the part to change in each treatment. Therefore, the holding device sufficiently securely holding a body part of a subject has not been developed yet at present.

The present invention was conceived in view of the situations and it is therefore an object of the present invention to provide a holding device sufficiently securely holding a body part of a subject.

### Solution to Problem

To achieve the above object, a first aspect of the present invention provides a holding device comprising: a holding tool including a curved surface formed in accordance with a shape of a body surface of a body part of a subject along with an adhesive layer having adhesiveness to a skin surface of the subject on the curved surface side; an elongated arm member having one end portion to which the holding tool is attached; and a fixing tool for fixing the arm member to a fixed object, attached to an end portion of the arm member on the side opposite to the end portion on the side to which the holding tool is attached.

### Advantageous Effects of the Invention

The first aspect of the present invention includes: the holding tool that includes the curved surface formed in accordance with a shape of a body surface of a body part of the subject and that includes the adhesive layer having adhesiveness to the skin surface of the subject on the side of the curved surface; the elongated arm member having the one end portion to which the holding tool is attached; and the fixing tool for fixing the arm member to the fixed object, attached to the end portion of the arm member on the side opposite to the end portion on the side to which the holding tool is attached, and therefore, for example, even in a treatment targeting an easily deforming part such as the breast, the holding tool can adhere to the part with the adhesive layer, so that the part can sufficiently securely be held. Thus, the holding device sufficiently securely holding a body part of the subject can be provided.

A second aspect of the present invention provides the holding device recited in the first aspect of the present invention, wherein the holding tool is a breast holding tool including the curved surface formed into a cup shape in accordance with a shape of a body surface including at least one breast of the subject along with the adhesive layer on the curved surface side. Consequently, the breast can sufficiently securely be held at the time of treatment for the breast easily deformed due to gravity depending on a posture etc. of the subject.

A third aspect of the present invention provides the holding device recited in the first or second aspect of the present invention, wherein the fixing tool is fixed to a bed having a flat plate portion on which the subject is placed. Consequently, a body part of the subject can sufficiently securely be held at the time of treatment performed while the subject is placed on the bed.

A fourth aspect of the present invention provides the holding device recited in any one of the first to third aspects of the present invention, wherein the arm member is capable of adjusting at least one of a position and an angle in a three-dimensional direction of the holding tool with respect to the fixing tool. Consequently, the position and the angle of the holding tool can appropriately be determined with respect to the fixed object.

A fifth aspect of the present invention provides the holding device recited in any one of the first to fourth aspects of the present invention, wherein the holding tool includes a plurality of hole portions penetrating in a thickness direction. Consequently, while the holding tool is attached to a body part of the subject, treatments such as collecting a biological tissue in the part and injecting a marker for radiation treatment into the part can be performed with a syringe, for example.

A sixth aspect of the present invention provides the holding device recited in any one of the first to fifth aspects of the present invention, wherein the holding tool is attachable to and detachable from the arm member, and wherein the holding tool matching a shape of a body surface on the body part of the subject is selected from a plurality of types of holding tools having a shape corresponding to the body part and formed into a plurality of sizes and is attached to the arm member before use. Consequently, the low-cost and practical holding device sufficiently securely holding the body part of the subject can be provided.

### BRIEF DESCRIPTION OF DRAWINGS

Fig. 1 is a plane view of how a holding device according to an embodiment of the present invention is attached to a fixed object.
Fig. 2 is a perspective view of an example of the configuration of the holding device according to the embodiment of the present invention.
Fig. 3 is a view of a configuration of the holding tool included in the holding device of Fig. 2, and a plane view of the holding tool from above.
Fig. 4 is a cross-sectional view taken along IV-IV of Fig. 3.
Fig. 5 is a perspective view of a configuration in which a holding tool having a form different from the holding tool shown in Figs. 3 and 4 is attached to the arm member.
Fig. 6 is a front view for detailed explanation of a configuration of a leading end portion provided on the arm member shown in Fig. 5.
Fig. 7 is a view of another form of attachment of the fixing tool to the fixed object of Fig. 1.
Fig. 8 is a view of another form of attachment of the holding tool to the arm member in the holding device of this embodiment.
Fig. 9 is a view of another form of the arm member included in the holding device of this embodiment.
Fig. 10 is a view of another form of the holding tool included in the holding device of this embodiment, and is a plane view of the holding tool from above.
Fig. 11 is a side view of the holding tool in the direction indicated by an arrow XI of Fig. 10.
Fig. 12 is a view of an example of a configuration of the holding tool including a plurality of hole portions, included in the holding device of this embodiment, and is a plane view of the holding tool from above.
Fig. 13 is a side view of the holding tool in the direction of an arrow XIII of Fig. 12.
Fig. 14 is a view of another example of the configuration of the holding tool formed into a mesh-like configuration and included in the holding device of this embodiment, and is a plane view of the holding tool from above.
Fig. 15 is a side view of the holding tool in the direction indicated by an arrow XV of Fig. 14.
Fig. 16 is a schematic side view of how the holding device including the holding tool shown in Figs. 10 to 15 is attached to the fixed object.
Fig. 17 is a plane view of a portion of the fixed object and the holding device in the direction indicated by an arrow XVII of Fig. 16.
Fig. 18 is a view of another form of the fixing tool included in the holding device of this embodiment.
Fig. 19 is a view of yet another form of the fixing tool included in the holding device of this embodiment.
Fig. 20 is a schematic cross-sectional view for explaining the effect of the holding device of this embodiment, showing how the holding tool is attached to a body part of the subject.
Fig. 21 is a schematic cross-sectional view for explaining the effect of the configuration of the holding tool provided with a plurality of hole portions, showing how the holding tool is attached to a body part of the subject.

### MODES FOR CARRYING OUT THE INVENTION

Preferred embodiments of the present invention will now be described in detail with reference to the drawings. The drawings used for the following description are not necessarily accurately drawn in terms of dimensional ratios etc. of portions. Constituent elements common to embodiments are denoted by the same reference numerals and will not be described.

### Embodiments

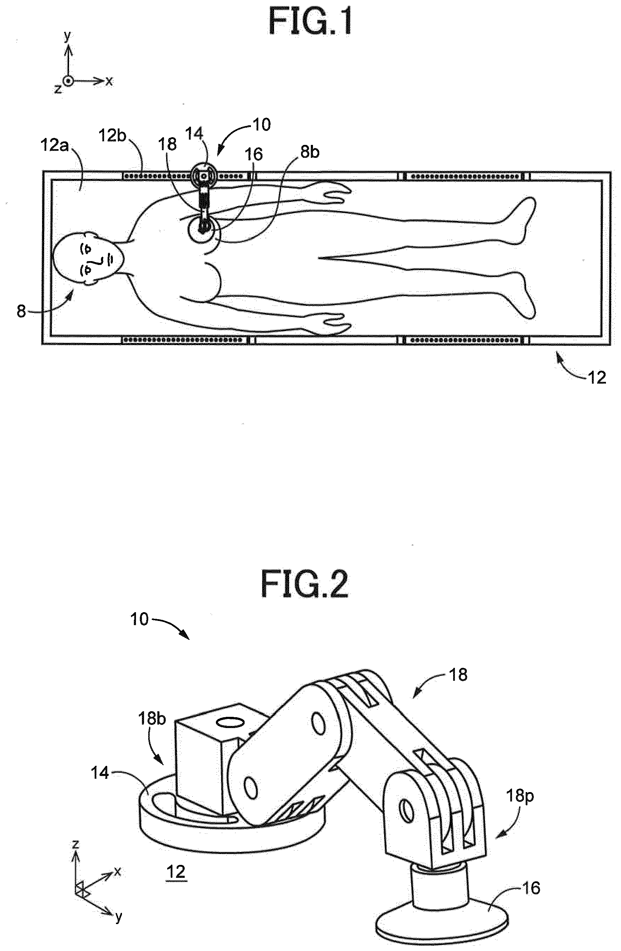

Fig. 1 is a view for explaining an example of a configuration of a holding device 10 that is a preferred embodiment of the present invention. The holding device 10 of this embodiment is, for example, a device holding a body part of a subject (patient) 8 at the time of treatment such as particle beam treatment, X-ray treatment, γ-ray treatment, electron beam treatment, CT imaging, and MRI imaging by a medical device not shown. As shown in Fig. 1, the holding device 10 is preferably attached to a bed 12 serving as a fixed object before use. For example, while the subject 8 is placed on a flat plate portion 12a of the bed 12 in a supine posture, a body part of the subject 8 is held with respect to the bed 12. The bed 12 is preferably supported by a supporting device not shown at the time of the treatment. More preferably, the bed 12 is transportable because of a stretcher not shown etc. to be used with.

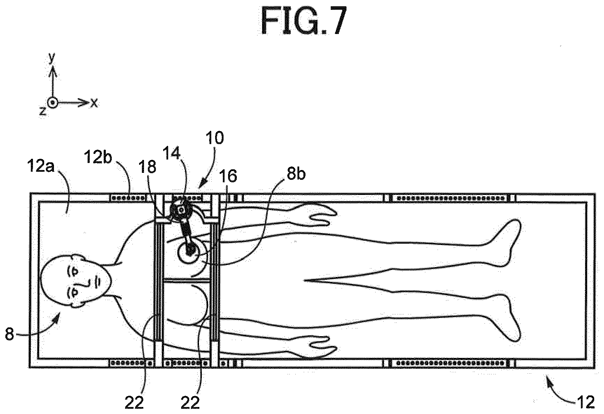

Fig. 2 is a perspective view of an example of the configuration of the holding device 10. As shown in Fig. 2, the holding device 10 includes a fixing tool 14, a holding tool 16, and an arm member 18. The fixing tool 14 is fixed to a fixed object that is an object to which the holding device 10 is attached. Preferably, the fixing tool 14 is fixed to the bed 12 serving as the fixed object. For example, the fixing tool 14 is fixed by a fastener such as a screw to an attaching portion 12b described later included in the bed 12. This form of fixation may be another form as described later with reference to Figs. 7, 18, 19, etc. Preferably, the fixing tool 14 is attachable to and detachable from the fixed object to which the holding device 10 is attached.

In the holding device 10, preferably, main portions of configurations of the fixing tool 14, the holding tool 16, and the arm member 18 are made of a synthetic resin material. The holding tool 16 is preferably formed of a synthetic resin material such as an acrylic resin, an ABS resin, and a polylactic resin. Alternatively, the holding tool 16 may be formed of a fabric member made of fiber such as glass fiber, polyester fiber, or cotton impregnated with a curing agent and cured in accordance with a shape of a body surface on the body part.

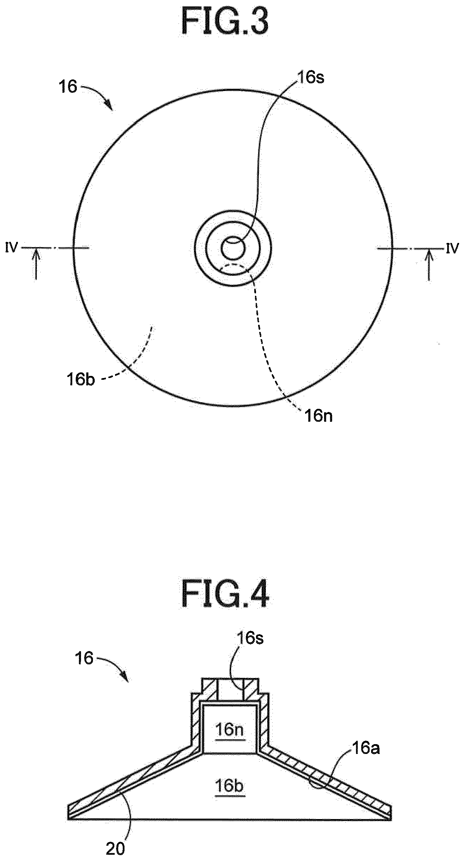

Figs. 3 and 4 are views of a configuration of the holding tool 16 included in the holding device 10, and Fig. 3 is a plane view of the holding tool 16 from above while Fig. 4 is a cross-sectional view taken along IV-IV of Fig. 3. The holding tool 16 includes a curved surface 16a formed in accordance with the shape of the body surface on the body part of the subject 8. The holding tool 16 includes an adhesive layer 20 having adhesiveness to a skin surface of the subject 8 on the curved surface 16a side thereof. The adhesive layer 20 is preferably provided to cover the curved surface 16a.

The holding tool 16 is preferably a breast holding tool including the curved surface 16a as an inner surface formed into a cup shape in accordance with the shape of the body surface including at least one breast 8b of the subject 8 along with the adhesive layer 20 disposed on the curved surface 16a side thereof. The holding tool 16 is formed into a shape partially or entirely covering at least the breast 8b including a papilla (nipple) of the subject 8. Therefore, preferably, as shown in Figs. 3 and 4, the holding tool 16 includes a space 16n receiving the papilla of the subject 8, and a space 16b partially or entirely receiving the breast 8b of the subject 8. More preferably, the holding tool 16 is formed into a shape partially or entirely covering a region including the breast 8b, the breastbone, the collarbone, and the ribs of the subject 8.

As shown in Fig. 4, the holding tool 16 includes the adhesive layer 20 having adhesiveness to the skin surface of the subject 8 on the side of the curved surface 16a that is an inner surface formed in accordance with the shape of the body surface including the breast 8b. Therefore, when the holding tool 16 is attached to the breast 8b of the subject 8, the adhesive layer 20 having adhesiveness to the skin surface of the breast 8b is included on an entire or partial surface of a part of the holding tool 16 coming into contact with the breast 8b. The adhesive layer 20 is preferably made of a resin material having adhesiveness such as urethane gel or silicone gel. For the adhesive layer 20, an acrylic-, silicone-, urethane-, or rubber-based adhesive material may be used. For the adhesive layer 20, a synthetic resin material having adhesiveness such as acrylic-, silicone-, urethane-, styrene-based elastomer may be used. As shown in Fig. 4, preferably, the holding tool 16 is provided with the adhesive layer 20 covering an inner circumferential surface of the space 16n receiving the papilla of the subject 8 and an inner circumferential surface of the space 16b partially or entirely receiving the breast 8b of the subject 8.

With regard to the holding tool 16, preferably, the holding tool 16 matching the shape of the body surface on the body part of the subject 8 is selected from a plurality of types of the holding tools 16 having a shape corresponding to the body part of the subject 8 and formed into a plurality of sizes and is attached to the arm member 18 before use. In particular, a plurality of types of the holding tools 16 including the curved surfaces 16a formed in accordance with shapes of body surfaces on body parts of subjects 8 is prepared in advance correspondingly to respective multiple forms expected for the shape of the body surface on the body part. At the time of treatment of the subject 8, the holding tool 16 matching the shape of the body surface on the body part of the subject 8 is selected and used out of the plurality of types of the holding tools 16. In this embodiment, from the plurality of types of the holding tools 16 formed into sizes and shapes corresponding to the shape of the breast 8b (a type of so-called cup, a difference between top-bust and under-bust, etc.), the holding tool 16 matching the shape of the body surface on the breast 8b of the subject 8 is appropriately selected and attached to the arm member 18 before use.

As shown in Fig. 2, the arm member 18 is an elongated member having one end portion to which the holding tool 16 is attached and the other end portion to which the fixing tool 14 is attached. The holding tool 16 is attached to the arm member 18 by fitting an attaching portion 16s (see Fig. 4 etc.) included in the holding tool 16 to a portion of a leading end portion 18p of the arm member 18, for example. The holding tool 16 is preferably attachable to and detachable from the arm member 18. The fixing tool 14 is attached to the arm member 18 at a base end portion 18b that is an end portion on the side opposite to the leading end portion 18p.

As shown in Fig. 1, the bed 12 includes the attaching portion 12b to which the fixing tool 14 is attached. Preferably, a pair or a plurality of pairs of the attaching portions 12b are provided to the right and left of the subject 8 placed on the bed 12 in a supine position. Each of the attaching portions 12b has a plurality of attaching positions arranged in a head-foot direction of the subject 8 placed on the bed 12 in a supine position such that the fixing tool 14 is selectively attached thereto. For example, multiple threaded holes for screwing a male screw related to attachment of the fixing tool 14 are arranged at substantially equal intervals. By attaching the fixing tool 14 to any of these attachment positions, the position of the base end portion 18b (see Fig. 2 etc.) of the arm member 18 is determined in the head-foot direction, i.e., the direction indicated by the x-axis of Fig. 1, with respect to the bed 12.

The arm member 18 has a multi-joint structure as shown in Fig. 2, for example, and is therefore configured such that at least one of the position and the angle in the three-dimensional direction of the holding tool 16 attached to the leading end portion 18p can be adjusted with respect to the base end portion 18b. Preferably, the arm member 18 is configured such that at least the position in the three-dimensional direction can be adjusted. Because of this configuration of the arm member 18, the holding device 10 enables adjustment of at least one of the position and the angle in the three-dimensional direction of the holding tool 16 with respect to the bed 12 while the fixing tool 14 is fixed to the bed 12 that is the fixed object. For example, when the head-foot direction of the subject 8 placed on the bed 12 in a supine position is defined as a reference direction, the holding device 10 enables the adjustment of at least one of the position and the angle in the three-dimensional direction of the holding tool 16 with respect to the bed 12. In particular, as shown in Fig. 1, when the head-foot direction of the subject 8 placed on the bed 12 in the supine position is used as the x-axis direction to define the γ-axis and the z-axis perpendicular to the x-axis and orthogonal to each other, the holding device 10 enables the adjustment of at least one of the position and the angle in the x-, γ-, or z-axis direction of the holding tool 16 with respect to the bed 12.

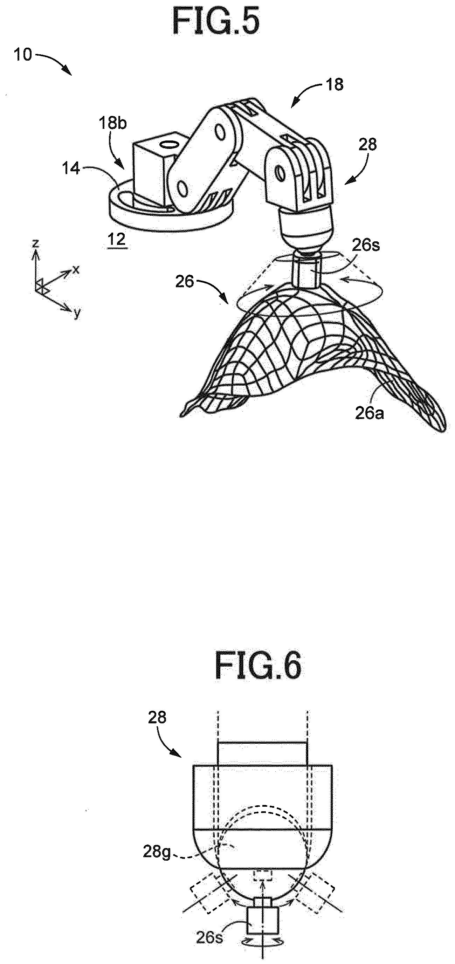

Fig. 5 is a perspective view of a configuration in which a holding tool 26 having a form different from the holding tool 16 is attached to the arm member 18. Fig. 6 is a front view for detailed explanation of a configuration of a leading end portion 28 provided on the arm member 18 and having a form different from the leading end portion 18p. The holding tool 26 is individually produced for each of the subjects 8 based on information related to the shape of the breast 8b of the subject 8 acquired by a known three-dimensional scanner, for example. For example, the holding tool 26 including a curved surface 26a corresponding to the shape of the breast 8b of the subject 8 is produced by a known 3D printer etc. based on the information. The curved surface 26a is formed into a shape partially or entirely covering a region including the breast 8b, the breastbone, the collarbone, and the ribs of the subject 8. The adhesive layer 20 having adhesiveness to the skin surface of the subject 8 is provided on the curved surface 26a side of the holding tool 26.

As shown in Fig. 6, a spherical body 28g is fitted inside the leading end portion 28. An attaching portion 26s of the holding tool 26 is attached to the spherical body 28g. With such a configuration, the holding tool 26 (the attaching portion 26s) can be adjusted to an arbitrary angle with respect to the leading end portion 28 of the arm member 18. Furthermore, as indicated by arrows of Fig. 6, the holding tool 26 (the attaching portion 26s) can rotate relatively to the leading end portion 28. Therefore, the angle of the holding tool 26 can be changed in the rotational direction around the attaching portion 26s with respect to the arm member 18. The holding member 16 described above with reference to Figs. 3 and 4 may be attached to the arm member 18 including the leading end portion 28 shown in Figs. 5 and 6.

Fig. 7 is a view of another form of attachment of the fixing tool 14 to the bed 12. In the configuration shown in Fig. 1 described above, the fixing tool 14 is directly attached to the attaching portion 12b provided on the bed 12; however, in the configuration shown in Fig. 7, a bridging portion 22 is attached to the bed 12 and the fixing tool 14 is fixed to the bridging portion 22. The bridging portion 22 is a pair of elongated members bridging between the paired attaching portions 12b provided to the right and left of the subject 8 placed on the bed 12 in the supine position. By attaching the bridging portion 22 to any of a plurality of attaching positions on the attaching portions 12b, the position of the bridging portion 22 is determined in the head-foot direction, i.e., the direction indicated by the x-axis of Fig. 7, with respect to the bed 12. The fixing tool 14 is fixed at an arbitrary position in the longitudinal direction of the bridging portion 22. By attaching the fixing tool 14 to a predetermined position in the longitudinal direction of the bridging portion 22, the position of the fixing tool 14 is determined in the direction perpendicular to the head-foot direction, i.e., the direction indicated by the γ-axis of Fig. 7, with respect to the bed 12. Therefore, in the configuration shown in Fig. 7, the fixing tool 14 is indirectly fixed via the bridging portion 22 to the bed 12, so that the position of the base end portion 18b in the arm member 18 can be determined in the x-axis direction and the γ-axis direction with respect to the bed 12 (i.e., the planar direction of the flat plate portion 12a).

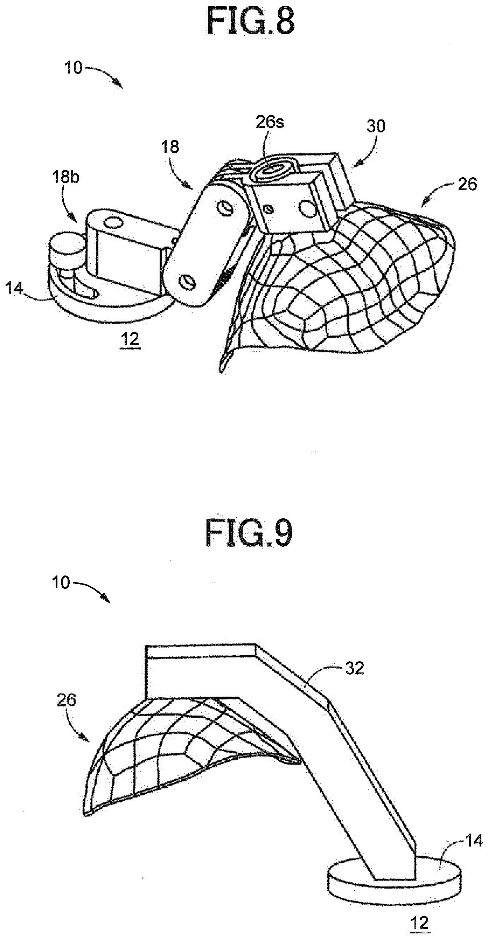

Fig. 8 is a view of another form of attachment of the holding tool 26 to the arm member 18. In the configuration shown in Fig. 8, the holding tool 26 has an attaching portion 26s sandwiched and fixed (clamped) by a leading end portion 30 of the arm member 18. In particular, the leading end portion 30 is provided with a gap into which the attaching portion 26s is inserted and, by fastening a fastener such as a screw so that the gap is narrowed while the attaching portion 26s is inserted in the gap, the attaching portion 26s is sandwiched and fixed by the leading end portion 30. The holding tool 16 may be attached to the arm member 18 including the leading end portion 30 shown in Fig. 8. In this configuration, the holding tools 16, 26, etc. can easily be attached to the arm member 18. Furthermore, according to the configuration shown in Fig. 8, since the leading end portion 30 is not located above the attaching portion 26s (on the side opposite to the holding tools 16, 26, etc. in the axial direction of the attaching portion 26s), an irradiation gantry etc. of a medical device not shown can be disposed above the attaching portion 26s. Therefore, as compared to the configuration described above with reference to Fig. 2 etc., the irradiation gantry etc. can be brought closer to an affected part to be treated (e.g., the breast 8b) of the subject 8, and the accuracy of treatment can be improved.

Fig. 9 is a view of another form of the arm member included in the holding device 10. In the configuration shown in Fig. 9, the holding device 10 includes an arm member 32 without a multi-joint structure. In particular, the arm member 32 is formed in a predefined elongated shape as a single unit. The holding tool 26 is attached to one end portion of the arm member 32 and the fixing tool 14 is attached to the other end portion of the arm member 32. The holding tool 16 may be attached to the arm member 32 shown in Fig 9. The arm member 32 can improve the positional fixation performance of the holding tools 16,26, etc. with respect to the bed 12. Additionally, the holding tools 16, 26, etc. and the arm member 18 can easily be attached to the bed 12. In the configuration shown in Fig. 9, preferably, the fixing tool 14, the holding tools 16, 26, etc., and the arm member 32 are formed as a single unit by a known 3D printer etc. in accordance with the shape of the affected part to be treated (e.g., the breast 8b), the lesion (tumor) position, the treatment plan, etc. of the subject 8 to be treated.

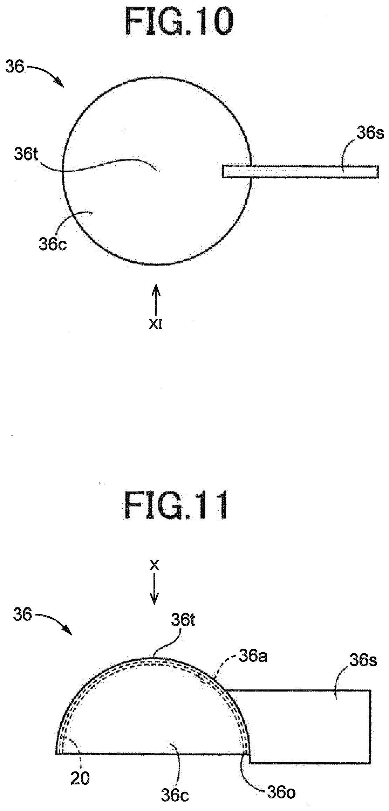

Figs. 10 and 11 are views of another form of the holding tool included in the holding device 10. Fig. 10 is a plane view of a holding tool 36 from above, and Fig. 11 is a side view of the holding tool 36 in the direction indicated by an arrow XI of Fig. 10. As shown in Figs. 10 and 11, the holding tool 36 includes a cup portion 36c formed into a cup shape and an attaching portion 36s formed into a flat plate shape. In Figs. 10 and 11, the hemispherical cup portion 36c is shown; however, the cup portion 36c may not necessarily have a hemispherical shape and is appropriately formed in accordance with the shape of the breast 8b. The cup portion 36c has a curved surface 36a as an inner surface formed into a cup shape in accordance with the shape of the body surface including at least the one breast 8b of the subject 8. As is the case with the holding tool 16, a space 16n etc. receiving the papilla of the breast 8b may be provided. As indicated by broken lines of Fig. 11, the holding tool 36 includes the adhesive layer 20 having adhesiveness to the skin surface of the subject 8 on the side of the curved surface 36a. The attaching portion 36s is provided on the side portion of the cup portion 36c. In particular, the attaching portion 36s is provided to project from the vicinity of a lower opening 360 (an end edge of the curved surface 20) of the cup portion 36c toward the outside (radial outside) of the cup portion 36c. In other words, the attaching portion 36s is provided at a position closer to the opening 360 than a top portion 36t of the cup portion 36c. Preferably, as is the case with the holding tool 16, the holding tool 36 matching the shape of the body surface on the body part of the subject 8 is selected from a plurality of types of the holding tools 36 having a shape corresponding to the body part of the subject 8 and formed into a plurality of sizes and is attached to an arm member 38 etc. described later before use.

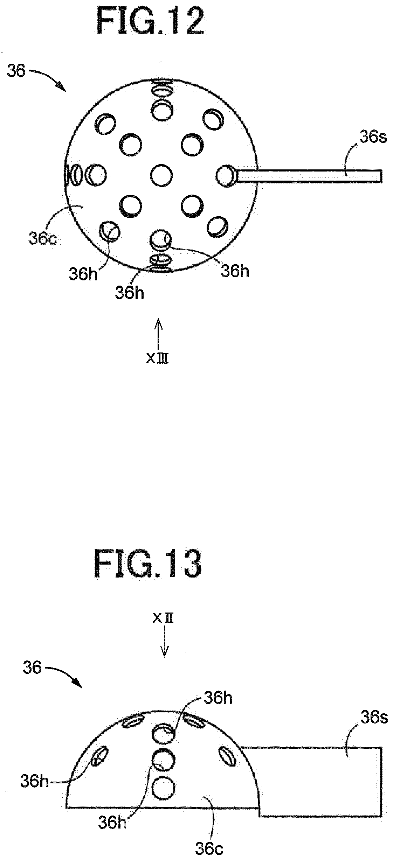

The holding tool 36 preferably includes a plurality of hole portions penetrating in the thickness direction. Figs. 12 and 13 are views of an example of a configuration of the holding tool 36 including a plurality of hole portions. Fig. 12 is a plane view of the holding tool 36 from above, and Fig. 13 is a side view of the holding tool 36 in the direction of an arrow XIII of Fig. 12. In the configuration shown in Figs. 12 and 13, the cup portion 36c includes a plurality of hole portions 36h penetrating in the thickness direction thereof. The hole portion 36h is provided to penetrate the adhesive layer 20 as well. Therefore, the adhesive layer 20 is not provided at positions corresponding to the hole portions 36h. In other words, when the holding tool 36 is attached to the breast 8b etc. of the subject 8, the skin surface of the breast 8b etc. is exposed at the positions corresponding to the hole portions 36h. Preferably, the hole portions 36h are formed into such a size that at least a needle of a typical syringe or a biopsy needle can be inserted, and are circular hole portions having a diameter dimension of 4 to 5 mmø or more, for example.

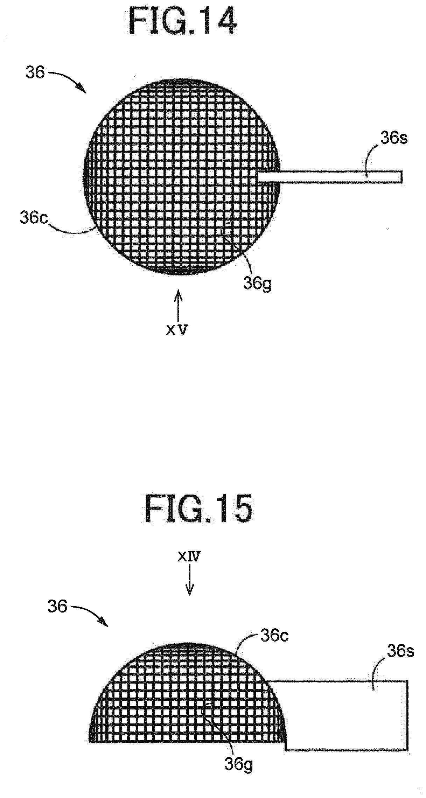

Figs. 14 and 15 are views of another example of the configuration of the holding tool 36 having a plurality of hole portions. Fig. 14 is a plane view of the holding tool 36 from above, and Fig. 15 is a side view of the holding tool 36 in the direction indicated by an arrow XV of Fig. 14. In the configuration shown in Figs. 14 and 15, the whole of the cup portion 36c is formed into a mesh-like configuration. Therefore, the cup portion 36c is formed into a sieve-like configuration. Gaps 36g of the mesh correspond to a plurality of hole portions penetrating the cup portion 36c in the thickness direction. The adhesive layer 20 is not provided at positions corresponding to the gaps 36g. When the holding tool 36 is attached to the breast 8b etc. of the subject 8, the skin surface of the breast 8b etc. is exposed at the positions corresponding to the gaps 36g. Preferably, the gaps 36g are formed into such a size that at least a needle of a typical syringe or a biopsy needle can be inserted, and are square gaps of 4 to 5 mm or more on a side, for example. The holding tool 36 shown in Figs. 14 and 15 preferably has the attaching portion 36s and the mesh-like cup portion 36c integrally formed by a known 3D printer etc. Alternatively, the holding tool 36 may be configured by fixedly attaching the attaching portion 36s formed of a synthetic resin material etc. to the cup portion 36c formed of a fabric member made of fiber such as glass fiber, polyester fiber, and cotton impregnated with a curing agent and cured in accordance with the shape of the breast 8b etc.

Although the configuration of the holding tool 36 including the plurality of hole portions 36h and having the mesh-like configuration have been described with reference to Figs. 12 to 15, the holding tool 16 described above with reference to Figs. 3 and 4 or the holding tool 26 described above with reference to Fig. 5 etc. may include a plurality of hole portions penetrating in the thickness direction. In particular, the holding tools 16, 26 may include a plurality of hole portions penetrating in the thickness direction as is the case with the holding tool 36 described above with reference to Figs. 12 and 13. The holding tools 16,26 may be formed into the mesh-like configuration over the whole of the holding tools 16, 26 as is the case with the holding tool 36 described with reference to Figs. 14 and 15.

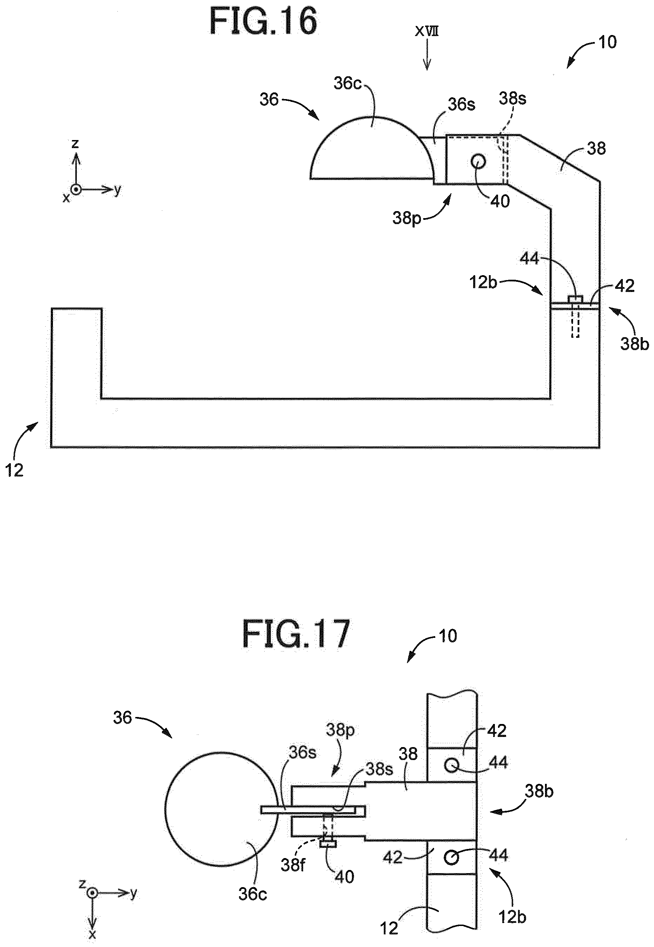

Figs. 16 and 17 are schematic views of how the holding device 10 including the holding tool 36 is attached to the bed 12 serving as the fixed object. Fig. 16 is a side view when the holding device 10 and the bed 12 are viewed in the head-foot direction, i.e., the direction indicated by the x-axis of Fig. 16. Fig. 17 is a plane view of a portion of the bed 12 and the holding device 10 in the direction indicated by an arrow XVII of Fig. 16. In Figs. 16 and 17, as with the arm member 32 described above with reference to Fig. 9, a configuration including the arm member 38 integrally formed without a multi-joint structure is exemplarily illustrated; however, the holding tool 36 is also preferably attached to an arm member formed into a multi-joint structure as in the case of the arm member 18 described above with reference to Fig. 2 etc. As shown in Fig. 17, a leading end portion 38p of the arm member 38 has a slit 38s formed with a width dimension allowing insertion of at least the attaching portion 36s of the holding tool 36. The leading end portion 38p has a threaded hole (female thread) 38f formed penetrating from a side thereof to the slit 38s. As shown in Fig. 17, a male screw 40 is fastened to the threaded hole 38f with the attaching portion 36s inserted in the slit 38s. As the male screw 40 is tightened, the attaching portion 36s is sandwiched between one inner wall of the slit 38s and a leading end of the male screw 40, so that the holding tool 36 is fixed to the arm member 38.

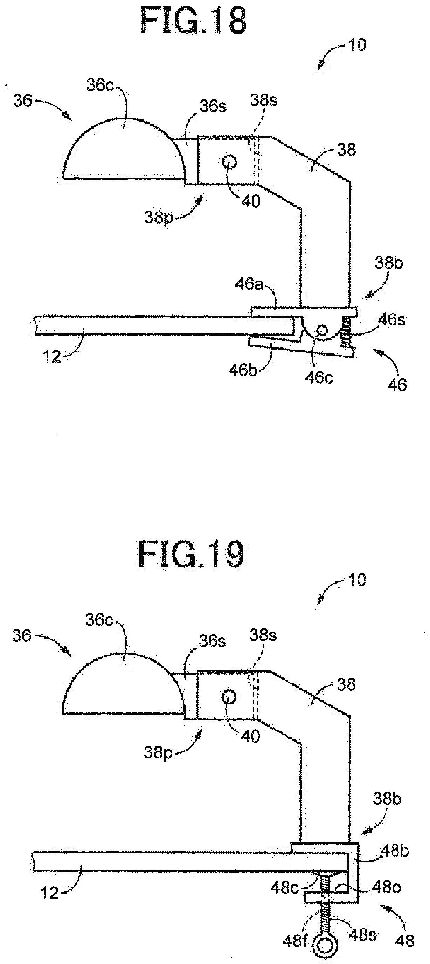

A base end portion 38b of the arm member 38 is provided with a flange portion 42 as a fixing tool. The flange portion 42 is preferably integrally formed as the same member as the arm member 38. As shown in Figs. 16 and 17, male screws 44 inserted through through-holes not shown formed in the flange portion 42 are fastened to threaded holes in the attaching portion 12b of the bed 12 so that the flange portion 42 is fixed to the bed 12. The holding device 10 having the configuration shown in Figs. 16 and 17 may be attached to the bed 12 in the form as shown in Fig. 7 described above. In particular, the bridging portion 22 may be attached to the bed 12 and the flange portion 42 serving as a fixing tool may be fixed to the bridging portion 22. Alternatively, as described in detail later with reference to Figs. 18 and 19, other forms of attachment of the holding device to the bed 12 are also available.

Fig. 18 is a view of another form of the fixing tool included in the holding device 10. As shown in Fig. 18, the holding device 10 may include a fixing tool in the form of a clip or the like 46 as an alternative to the flange portion 42 etc. In particular, in the configuration shown in Fig. 18, the fixing tool 46 is attached to the base end portion 38b of the arm member 38. The fixing tool 46 includes a first member 46a and a second member 46b. The first member 46a is fixed to the base end portion 38b of the arm member 38. The first member 46a and the second member 46b are provided relatively pivotally around a pivot shaft 46c. The first member 46a and the second member 46b are configured such that the fixed object such as the bed 12 can be sandwiched at one end portion of the fixing tool 46. At the other end portion of the fixing tool 46, a spring 46s is provided between the first member 46a and the second member 46b. When the fixed object is sandwiched between the first member 46a and the second member 46b in the fixing tool 46 configured in this way, the fixed object is grasped between the first member 46a and the second member 46b due to the elasticity of the spring 46s and the fixing tool 46 is fixed to the fixed object. Although Fig. 18 exemplarily illustrates a configuration of the fixing tool 46 attached to the arm member 38, the fixing tool 46 may be attached to the arm member 18 described above with reference to Figs. 2, 8, etc., and the device may be fixed by the fixing tool 46 to the fixed object.

Fig. 19 is a view of yet another form of the fixing tool included in the holding device 10. As shown in Fig. 19, the holding device 10 may include a fixing tool in the form of a vise or the like 48 as an alternative to the flange portion 42 etc. In particular, in the configuration shown in Fig. 19, the fixing tool 48 is attached to the base end portion 38b of the arm member 38. The fixing tool 48 includes a base portion 48b having a mouth portion 480 with dimensions at least allowing insertion of the fixed object and a screw portion 48s mainly made up of a male screw. The base portion 48b has a threaded hole (female thread) 48f formed penetrating from a lower side (an end portion on the side opposite to the side to which the arm member 38 is attached) to the mouth portion 480. The male screw of the screw portion 48s is threadably engaged with the threaded hole 48f. As shown in Fig. 19, when the male screw of the screw portion 48s is screwed into the threaded hole 48f with the fixed object such as the bed 12 inserted in the mouth portion 480, the fixed object is sandwiched between one inner wall (upper inner wall) of the mouth portion 480 and a receiving portion 48c provided at an leading end of the screw portion 48s, and the fixing tool 48 is fixed to the fixed object. Although Fig. 19 exemplarily illustrates a configuration of the fixing tool 48 attached to the arm member 38, the fixing tool 48 may be attached to the arm member 18 described above with reference to Figs. 2, 8, etc., and the device may be fixed by the fixing tool 48 to the fixed object.

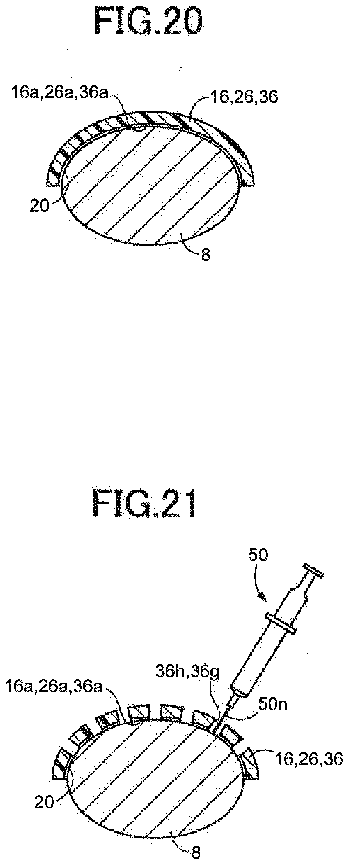

Fig. 20 is a schematic cross-sectional view for explaining the effect of the holding device 10 of this embodiment, showing how the holding tools 16, 26, 36 (hereinafter referred to as the holding tool 16 etc.) are attached to a body part of the subject 8. For example, in the particle beam treatment including proton beam treatment and heavy particle beam treatment, the body position of the subject 8 must appropriately be changed between the supine position, the lateral position, the prone position, etc., and additionally, since a relative positional relationship between a device irradiating a particle beam and the body of the subject 8 relates to determination of a treatment part, it is required to securely hold (fix) the body of the subject 8. Particularly, in the particle beam treatment for the breast 8b of the subject 8, the breast 8b must be placed away from major organs such as the heart and the lungs located near the breast 8b so as not to cause adverse effects on the major organs due to the irradiation of the particle beam. Since the breast 8b is easily affected by gravity and easily deformed in shape, the breast shape must be retained in a required body posture so as not to change the shape of the breast 8b during the treatment. Furthermore, in the particle beam treatment etc., it may be required to keep a part such as the breast 8b to be treated in substantially the same shape in a plurality of treatments performed at a time interval. The holding tool 16 etc. included in the holding device 10 of this embodiment have the configuration described above with reference to Figs. 1 to 19 and therefore allow the adhesive layer 20 to adhere to the skin surface of the part as shown in Fig. 20 while being attached to the part such as the breast 8b. Thus, even when the holding tool 16 etc. are once detached from the part such as the breast 8b and then attached again to the part, the part can be held in the same shape as the previous time.

Fig. 21 is a schematic cross-sectional view for explaining the effect of the configuration of the holding tool 16 etc. provided with a plurality of hole portions, showing how the holding tool 16 etc. are attached to a body part of the subject 8. For example, in biological tissue diagnosis (biopsy), it may be required to stick (insert) a needle of a syringe or a biopsy needle into an affected part so as to collect a diseased tissue (cell) in the affected part. Also in radiation treatment, it may be required to stick a needle of a syringe or a biopsy needle into an affected part so as to inject a marker into the affected part. As described above with reference to Figs. 12 to 15, in the configuration of the holding tool 16 etc. provided with a plurality of the hole portions 36h or the gaps 36g serving as hole portions (hereinafter referred to as the hole portions 36 etc.), as shown in Fig. 21, while the holding tool 16 etc. are attached to a body part (such as the breast 8b) considered as the affected part of the subject 8, a needle 50n of a syringe 50 can be inserted from the hole portions 36h etc. to stick the needle 50n into the body part. Therefore, while the holding tool 16 etc. are attached to the affected part, a diseased tissue in the affected part can be collected by the syringe 50. While the holding tool 16 etc. are attached to the affected part, a marker for radiation treatment can be injected by the syringe 50 into the affected part. Particularly, the holding tool 36 described above with reference to Figs. 12 to 15 has the attaching portion 36s provided on the side portion of the cup portion 36c and, therefore, for example, while the holding tool 36 is attached to the breast 8b, the needle 50n of the syringe 50 can be inserted in a relatively wide range including the periphery of the papilla.

This embodiment includes: the holding tools 16, 26, 36 that include the curved surfaces 16a, 26a, 36a formed in accordance with a shape of a body surface of a body part of the subject 8 and that include the adhesive layer 20 having adhesiveness to the skin surface of the subject 8 on the side of the curved surfaces 16a, 26a, 36a; the elongated arm members 18, 32, 38 having one end portion to which the holding tools 16, 26, 36 are attached; and the fixing tools 14, 46, 48, for fixing the arm members 18, 32, 38 to the bed 12 etc. serving as the fixed object, or the flange portion 42 serving as the fixing tool, attached to end portions of the arm members 18, 32, 38 on the side opposite to the end portion on the side to which the holding tools 16, 26, 36 are attached, and therefore, for example, even in a treatment targeting an easily deforming part such as the breast 8b, the holding tools 16, 26, 36 can adhere to the part with the adhesive layer 20, so that the part can sufficiently securely be held. Thus, the holding device 10 sufficiently securely holding a body part of the subject 8 can be provided.

Since the holding tools 16, 26, 36 are breast holding tools including the curved surfaces 16a, 26a, 36a formed into a cup shape in accordance with the shape of the body surface including at least the one breast 8b of the subject 8 along with the adhesive layer 20 on the side of the curved surfaces 16a, 26a, 36a, the breast 8b can sufficiently securely be held at the time of treatment for the breast 8b easily deformed due to gravity depending on a posture etc. of the subject 8.

Since the fixing tools 14, 46, 48 or the flange portion 42 serving as the fixing tool is fixed to the bed 12 having the flat plate portion 12a on which the subject 8 is placed, a body part of the subject 8 can sufficiently securely be held at the time of treatment performed while the subject 8 is placed on the bed 12.

Since the arm member 18 is capable of adjusting at least one of a position and an angle in a three-dimensional direction of the holding tools 16, 26, 36 with respect to the fixing tool 14, the position and the angle of the holding tools 16, 26, 36 can appropriately be determined with respect to the fixed object.

Since the holding tools 16, 26, 36 include a plurality of the hole portions 36h penetrating in the thickness direction or the gaps 36g serving as the hole portions, while the holding tools 16, 26, 36 are attached to a body part of the subject 8, treatments such as collecting a biological tissue in the part and injecting a marker for radiation treatment into the part can be performed with the syringe 50, for example.

Since the holding tools 16, 36 are attachable to and detachable from the arm members 18, 32, 38, and the holding tools 16, 36 matching a shape of a body surface on the body part of the subject 8 are selected from a plurality of types of the holding tools 16, 36 having a shape corresponding to the body part and formed into a plurality of sizes and are attached to the arm members 18, 32, 38 before use, the low-cost and practical holding device 10 sufficiently securely holding the body part of the subject 8 can be provided.

Although the preferred embodiments of the present invention have been described in detail with reference to the drawings, the present invention is not limited thereto and may be implemented in other forms.

For example, although the arm member 18 having a multi-joint structure as shown in Fig. 2 and the arm members 32, 38 integrally formed without a joint are exemplarily illustrated in the embodiments, the arm member included in the holding device 10 of the present invention is not limited thereto, and an arm member in another form may be used. For example, although the arm member 18 including two joints is exemplarily illustrated in Fig. 2, the arm member of the present invention may include one joint (single joint) or three or more joints. A known flexible arm etc. may be used as a substitute for the arm member 18 etc. as long as the position, the angle, etc. of the holding tool 16 etc. can sufficiently securely be fixed with respect to the fixed object. The fixing tool 14 etc. may include a portion configured in an elongated shape and the portion configured in an elongated shape may substantially function as a portion of the arm member. For example, an arm member may be connected via a joint to a leading end of the portion configured in an elongated shape in the fixing tool 14. It is also conceivable that the holding tool 26 and the arm member 32 etc. integrally formed as described above with reference to Fig. 9 are connected via a joint to the leading end of the portion configured in an elongated shape in the fixing tool 14. Therefore, the holding device of the present invention may be any device substantially (functionally) including a configuration corresponding to the holding tool 16 etc., the arm member 18 etc., and the fixing tool 14 etc. described in the embodiments, and the detailed forms thereof are not limited to the configurations in the embodiments described above.

In the embodiments described above, the bed 12 including the flat plate portion 12a for placing the subject 8 thereon is exemplarily illustrated as the fixed object to which the fixing tool 14 etc. are fixed; however, this is not a limitation of the fixed object to which the fixing tool 14 etc. are fixed, and various forms are conceivable. For example, the fixing tool 14 etc. may be fixed to a stand such as a tripod serving as the fixed object. The fixing tool 14 etc. may be fixed to a wall of a room in which a medical device giving treatment to the subject 8 is disposed, or to the medical device itself.

Although the embodiments have been described in detail in terms of the effects when the holding device 10 is used at the time of the particle beam treatment including proton beam treatment and heavy particle beam treatment, the biological tissue diagnosis, and the radiation treatment; however, the holding device of the present invention is obviously not limited to these use applications. For example, also in the case of holding a part to be treated at the time of electron beam treatment and γ-ray treatment etc., the effect of the present invention can be acquired by holding the part with the holding device 10. Also in the case of holding a part to be treated at the time of CT imaging, MRI imaging, etc., the effect of the present invention can be acquired by holding the part with the holding device 10.

Although the holding device 10 holding the breast 8b of the subject 8 has been described in the embodiments, the object to be held by the holding device of the present invention is not limited to the breast 8b of the subject 8. For example, it is conceivable that at the time of treatment of skin cancer including melanoma (malignant melanoma), X-rays etc. are irradiated to the part while stretching out (pulling) the skin in which the skin cancer exists in the body of the subject. The holding tool included in the holding device of the present invention has the adhesive layer having adhesiveness to the skin surface of the subject on the curved surface side and is therefore preferably used even when the skin of the subject is stretched out as described above, and can sufficiently securely hold the part.

Although not exemplarily illustrated one by one, the present invention is implemented with other various modifications without departing from the spirit thereof.

### REFERENCE SIGNS LIST

8: Subject 8b: Breast (Body part) 10: Holding device 12: Bed (Fixed object) 12a: Flat plate portion 14, 46, 48: Fixing tool 16, 26, 36: Holding tool 16a, 26a, 36a: Curved surface 18, 32, 38: Arm member 20: Adhesive layer 36g: Gap (Hole portion) 36h: Hole portion 42: Flange portion (Fixing tool)

## Claims

1. A holding device comprising:
a holding tool including a curved surface formed in accordance with a shape of a body surface of a body part of a subject along with an adhesive layer having adhesiveness to a skin surface of the subject on the curved surface side;
an elongated arm member having one end portion to which the holding tool is attached; and
a fixing tool for fixing the arm member to a fixed object, attached to an end portion of the arm member on the side opposite to the end portion on the side to which the holding tool is attached.

2. The holding device according to claim 1, wherein
the holding tool is a breast holding tool including the curved surface formed into a cup shape in accordance with a shape of a body surface including at least one breast of the subject along with the adhesive layer on the curved surface side.

3. The holding device according to claim 1 or 2, wherein
the fixing tool is fixed to a bed having a flat plate portion on which the subject is placed.

4. The holding device according to any one of claims 1 to 3, wherein
the arm member is capable of adjusting at least one of a position and an angle in a three-dimensional direction of the holding tool with respect to the fixing tool.

5. The holding device according to any one of claims 1 to 4, wherein
the holding tool includes a plurality of hole portions penetrating in a thickness direction.

6. The holding device according to any one of claims 1 to 5, wherein
the holding tool is attachable to and detachable from the arm member, and wherein
the holding tool matching a shape of a body surface on the body part of the subject is selected from a plurality of types of holding tools having a shape corresponding to the body part and formed into a plurality of sizes and is attached to the arm member before use.
